# EUROPEAN PATENT SPECIFICATION

(11) **EP 0 579 331 B1**
(45) Date of publication and mention of the grant of the patent: **12.05.1999**
(21) Application number: 93202064.7
(22) Date of filing: 13.07.1993
(51) Int. Cl.: G01N 33/84

(54) **Dry analytical element and method for the colorimetric determination of lithium ion**
Trockenes, analytisches Element und Methode zur kolorimetrischen Bestimmung von Lithiumionen
Elément sec analytique et méthode de détermination colorimétrique d'ions de lithium

(30) Priority: 17.07.1992 US 916248
(43) Date of publication of application: 19.01.1994
(73) Proprietor: Johnson & Johnson Clinical Diagnostics, Inc., Rochester New York 14650 (US)
(72) Inventor: Schaeffer, James Robert, c/o EASTMAN KODAK COMPANY, Rochester, New York 14650-2201 (US); Winterkorn, Robert Francis, c/o EASTMAN KODAK CO., Rochester, New York 14650-2201 (US); Arter, Thomas Charles, c/o EASTMAN KODAK COMPANY, Rochester, New York 14650-2201 (US)
(74) Representative: Mercer, Christopher Paul

(56) References cited:
- EP-A- 0 516 226
- EP-A- 0 516 227
- US-A- 2 835 579

## Description

The present invention relates to clinical chemistry. In particular, it relates to a multilayer analytical element and method for its use in the colorimetric determination of lithium ion in aqueous specimens, such as biological fluids.

Lithium, in the form of lithium carbonate, is administered to manic-depressive patients. However, the therapeutic range of lithium ion in plasma is quite narrow, and thus it is very important to accurately monitor the level of lithium ion in such patients because of the toxic side effects that appear when it exceeds the therapeutic level. Such determinations are generally made in the presence of a number of other ions, such as sodium and potassium ions, in the test specimen.

A number of analytical reagents and methods have been devised to detect lithium ion colorimetrically in the presence of other ions. For example, US-A-5,051,367 describes the use of tetra-substituted aryl cyclic formazan dyes for this purpose in either solution or dry assay formulations.

More recently, a dry multilayer analytical element for a colorimetric lithium ion assay is described in EP-A-0 516 227 which was published after the present application's priority date. The important advance provided by the invention in that application is the use of certain 14-crown-4-ether derivative dyes in certain reagent layers of the elements to overcome problems with interferents. Such dyes are described in considerable detail in EP-A-0 516 227.

One embodiment of the multilayer element described above has a first reagent layer containing the 14-crown-4-ether derivative dye and an aromatic coupler solvent, such as 2,4-di-n-pentylphenol. The element also contains a second reagent layer, a subbing layer and an outermost porous spreading layer. Coupler solvent is in the first reagent layer only.

Even with the significant improvement represented by the multilayer element described above, there is additional need for improvement in the precision of lithium ion determination. Moreover, a reduction of the background rate of dye development is desired. Background rate refers to dye developed with time which is not related to the presence of lithium ion. Thus, an improved element for lithium ion determination is desired.

We have overcome the problems noted above and further improved a method for colorimetric determination of lithium ion using a dry multilayer analytical element for the determination of lithium ion, the element comprising a support having thereon, in order and in fluid contact:
a) a first reagent layer containing a 14-crown-4-ether derivative dye for lithium ion,
b) a second reagent layer containing a perfluoroalkyl surfactant, and an aromatic phenol organic solvent having the structure (I): 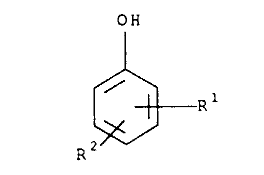 wherein R¹ and R² are independently alkyl of 1 to 20 carbon atoms or acyl of 2 to 20 carbon atoms and
c) a porous spreading layer, the element further comprising, in a layer other than the first or second reagent layers, a buffer present in an amount sufficient to maintain a pH in the element of at least 9 during an assay,
the element characterized wherein it further comprises a second aromatic phenol organic solvent in the first reagent layer, which second aromatic phenol organic solvent has the structure (I), and the first and second organic solvents can be the same or different and wherein in at least the dry state 14-crown-4 ether derivative dye for lithium ion is absent from the second reagent layer.

This invention also provides a method for the determination of lithium ion which comprises:
A. contacting a liquid sample suspected of containing lithium ion with the dry multilayer analytical element described above, and
B) determining the change in spectrophotometric signal in the element from complexation of the 14-crown-4-ether derivative dye with lithium ion in the liquid sample.

The present invention provides a dry analytical element for the effective and specific detection of lithium ion in a relatively short period of time using a colorimetric signal producing chemistry. Background rate of dye development is reduced with the present invention. Thus, the time for evaluating the colorimetric signal can be lengthened without losing precision, thereby providing the advantage of more accurate determination of lithium ion concentration in a narrow therapeutic range. This improvement is achieved by incorporating an aromatic phenol organic solvent in both the first and second reagent layers which are both below the porous spreading layer. The previous element (of EP-A-0 516 227) contained an organic solvent in the bottom reagent layer only. Putting it into both reagent layers unexpectedly improved the precision, by reducing the background rate of dye development. Further advantage is achieved by having a perfluoroalkyl surfactant in at least the second reagent layer as defined herein.

FIGURE 1 is a graphical plot of reflectance density (D_{R}) versus time (minutes) resulting from assay of lithium ion using a Control element and several calibrator fluids as described in Example 5 below.

FIGURE 2 is a graphical plot of reflectance density (D_{R}) versus time (minutes) resulting from assay of lithium ion using an element of this invention and several calibrator fluids as described in Example 5 below.

FIGURE 3 is a graphical plot of reflectance density(D_{R}) versus time (minutes) resulting from assay of lithium ion using a Control element as described in Example 6 below.

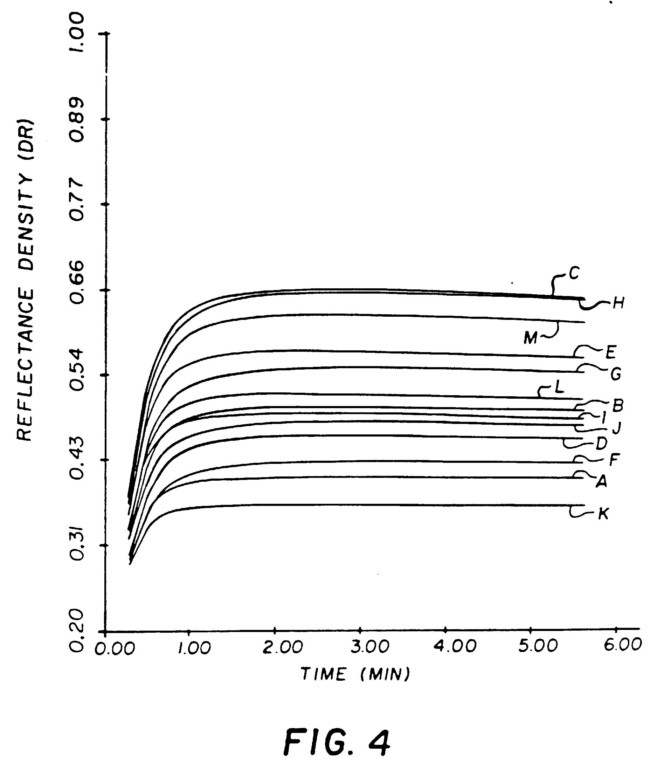

FIGURE 4 is a graphical plot of reflectance density(D_{R}) versus time (minutes) resulting from assay of lithium ion using an element of this invention as described in Example 6 below.

The element of this invention can be used to determine (that is, detect either the presence, amount or both) lithium ion in biological fluids of animals or humans, but preferably in humans. Such fluids include, but are not limited to, whole blood, plasma, sera, lymph, bile, urine, semen, cerebral spinal fluid, spinal fluid, sputum, perspiration, synovial fluid, lacrimal fluid and stool specimens as well as other biological fluids readily apparent to one skilled in the art. Fluid preparations of tissues can also be assayed. Preferably, human serum is assayed with this invention.

In its broadest embodiment, the dry element of this invention has an inert support with two reagent layers and a porous spreading layer disposed thereon. The support is generally dimensionally stable, inert to chemical reaction and preferably transparent (that is, radiation transmissive for wavelengths between 200 and 900 nm). However, non-transparent supports can be used if the mode of detection is reflectance spectroscopy instead of transmission spectroscopy. Useful supports are well known in the art, and include but are not limited to polyesters, papers, metal foils and polystyrene, polycarbonates and cellulose esters.

The porous spreading zone is prepared from any of the known materials used for such zones as described, for example in US-A-4,292,272, US-A-3,992,158, US-A-4,258,001, US-A-4,430,436 and JP 57(1982)-101760.

Preferred spreading zones are those described in US-A-3,992,158 as "blush polymer" zones. Such zones can be formed on a supporting material by dissolving a polymer in a mixture of two organic liquids, one of which is a lower boiling, good solvent for the polymer and other being a high boiling, non-solvent or poor solvent for the polymer. Within the porous zone can be incorporated particulate materials of various sizes to enhance the void volume.

The elements have at least two other layers which can contain one or more reagents needed for the assay. Such a layer can also be a second porous spreading layer, if desired. All of the layers are generally in fluid contact with each other, meaning that fluids, reagents and reagent products can pass or be transported between superposed regions of adjacent layers, unless of course, a reagent is immobilized in some manner so it will not migrate within or without a layer.

The reagent layers can be composed of one or more binder materials [such as gelatin and other colloidal materials, hydrophilic polymers such as poly(vinyl alcohol), acrylamide polymers, vinylpyrrolidone polymers and others known in the art] in which reagents are incorporated.

In a first reagent layer nearest the support, is a 14-crown-4-ether derivative which is reactive with lithium ion to provide a colorimetric signal. Such a signal can be a shift in absorbance from invisible to visible (to the human eye), visible to invisible, or from one visible wavelength to another. A number of such derivatives are known in the art, for example JP 62/72683, JP 62/56485 and EP-A-0 516 227. The preferred derivatives are those represented by the structure (II): 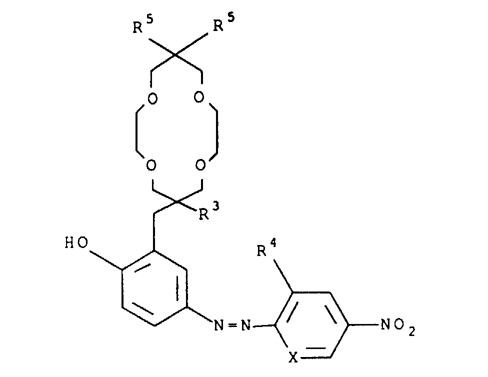 wherein R³ is substituted or unsubstituted alkyl of 1 to 15 carbon atoms (for example, methyl, ethyl, isopropyl, n-butyl, t-butyl, pentyl, hexyl, octyl, decyl, dodecyl, 3-ethyl-5-methyloctyl, benzyl and benzyl substituted with one or more lower alkyl groups such as methyl, ethyl and isopropyl). Also, R⁴ is hydrogen, nitro, methylsulfonyl, N-alkylsulfamoyl or N,N-dialkylsulfamoyl wherein the alkyl is substituted or unsubstituted and has 1 to 20 carbon atoms (such as methyl, ethyl, isopropyl, hexyl, benzyl, decyl, dodecyl, octadecyl and eicosyl). R⁵ is substituted or unsubstituted alkyl of 1 to 10 carbon atoms (for example, methyl, ethyl, isopropyl, isobutyl, n-butyl, heptyl and decyl), and X is N or CH.

More preferred compounds of structure (II) are those wherein R³ is methyl, benzyl or dodecyl, R⁴ is hydrogen, nitro, methylsulfonyl or sulfamoyl, R⁵ is methyl or heptyl and X is CH. Representative compounds are those in the following Table I with Compound 2 being most preferred:

**TABLE I**

| | R³ | R⁴ | R⁵ | X |
|---|---|---|---|---|
| 1 | n-C₁₂H₂₅ | SO₂CH₃ | CH₃ | CH |
| 2 | n-C₁₂H₂₅ | NO₂ | CH₃ | CH |
| 3 | CH₃ | SO₂N(n-C₆H₁₃)₂ | CH₃ | CH |
| 4 | CH₂C₆H₅ | SO₂N(n-C₆H₁₃)₂ | CH₃ | CH |
| 5 | CH₂C₆H₅ | SO₂N(CH₃)-n-C₁₈H₃₇ | CH₃ | CH |
| 6 | CH₃ | NO₂ | n-C₇H₁₅ | CH |
| 7 | n-C₁₂H₂₅ | H | CH₃ | N |

Also present in the reagent layer containing the 14-crown-4-ether derivative is an aromatic phenol organic solvent having the structure (I): 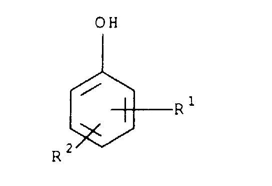 wherein R¹ and R² are independently alkyl of 1 to 20 carbon atoms (such as methyl, ethyl, isopropyl, n-butyl, t-butyl, n-pentyl, t-pentyl, hexyl, octyl, decyl, dodecyl and eicosyl) or acyl of 2 to 20 carbon atoms (such as acetyl, propionyl, valeryl and butyryl). Preferably, at least one of R¹ and R² is alkyl, and the groups are in the 2- and 4-positions in relation to the hydroxy group.

More preferably, R¹ and R² are independently alkyl of 1 to 10 carbon atoms, or acyl of 3 to 6 carbon atoms. Representative aromatic phenol organic solvents include those described in Research Disclosure, March, 1978, pages 23-24, and US-A-2,835,579. Particularly useful solvents include 2,4-di-n-pentylphenol, 2,4-di-t-pentylphenol, 2-n-octyl-4-pentylphenol, octyl-4-pentylphenol, 2-n-nonyl-4-n-pentylphenol, methyl-4-n-decylphenol, 2-n-decyl-4-methylphenol, and 2-n-valeryl-4-n-pentylphenol. Other useful solvents would be readily apparent to one skilled in the art. Most preferred is 2,4-di-n-pentylphenol.

A second reagent layer is disposed on the first reagent layer. The two layers can be immediately adjacent, or separated by a thin subbing layer (described below) as long as the reagents and fluid can readily move between the layers. In this second reagent layer is an aromatic phenol organic solvent which also is defined by structure (I). The solvents in the two reagent layers can be the same or different, but preferably, they are the same compound. A mixture of aromatic phenol organic solvents can be used in either layer if desired.

Optionally, but preferably, the porous spreading layer is separated from the two reagent layers with a hydrophilic subbing layer composed of one or more suitable hydrophilic binder materials. Such materials include, but are not limited to gelatin and other colloidal materials, polymers of vinyl pyrrolidone, vinyl alcohol, acrylamide, N-alkyl-substituted acrylamide (such as N-isopropylacrylamide), including copolymers thereof, and other materials readily apparent to one skilled in the art.

The pH of at least 9 in the element is maintained during an assay for lithium ion by one or more suitable buffers. The buffer is incorporated into one or more layers other than the two reagent layers, and preferably, it is in the hydrophilic subbing layer. Useful buffers include, but are not limited to, 3-(cyclohexylamino)-1-propanesulfonic acid, 4-aminobutyric acid, phosphate and arginine. Preferably, the pH is maintained at 10 or above with the upper limit being 14.

It is particularly useful to have one or more perfluoroalkyl surfactants in at least the second reagent layer. Preferably, such a surfactant is placed in both reagent layers. Such surfactants can be anionic, cationic, nonionic or amphoteric in nature. They have one or more fluorocarbon moieties in the molecule, each of which has one or more hydrogen atoms replaced by a fluorine atom. Each fluorocarbon moiety has at least 4 carbon atoms, and can be saturated or unsaturated. When the surfactant has two or more fluorocarbon moieties, each moiety are predominantly straight chain groups having no more than one or two small branches (for example, methyl or ethyl, fluorinated or unfluorinated). The moieties can be connected together by an organic backbone which is linear or branched. When the surfactant has only one fluorocarbon moiety, that moiety can be branched or linear.

More particularly, each fluorocarbon moiety has from 4 to 16 carbon atoms, from 8 to 33 fluorine atoms, and a ratio of fluorine atoms to carbon atoms of at least 2:1.

Representative surfactants are as follows:

### Nonionic Surfactants:

Perfluoroalkylpoly(ethylene (ethylene oxide) alcohols, for example, commercially available as ZONYL^{TM} FSN from DuPont, or as FLUOWET^{TM} OT from American Hoechst, or ethoxylated perfluoroalkyl materials such as SURFLON^{TM} 145 from Asahi Glass (also known as "surfactant F-35").

### Cationic Surfactants:

Perfluoroalkyl quaternary ammonium salts, for example, commercially available as FLUORAD^{TM} FC 135 from 3M Corporation, as LODYNE^{TM} 106 from Ciba-Geigy, or as ZONYL^{TM} FSC from DuPont.

### Amphoteric Surfactants:

Perfluoroalkyl betaines, for example, commercially available as SURFLON^{TM} S-132 from Asahi Glass Co., or as ZONYL^{TM} FSK from DuPont.

### Anionic Surfactants:

Fluoroalkylsulfates, for example commercially available as FLUORTENSID^{TM} FT-248 from Bayer, or as FLUOWET^{TM} SB from American Hoechst, or fluoroalkylalkylenethioalkylenecarboxylates, for example commercially available as ZONYL^{TM} FSA from DuPont.

Preferred perfluoroalkyl surfactants include the perfluoroalkyl betaines (such as SURFLON^{TM} S-132 amphoteric surfactant) and the ethoxylated perfluoroalkyl surfactants (such as SURFLON^{TM} S-145).

The amount of useful perfluoroalkyl surfactant in each reagent layer is generally at least 0.005 g/m² with an amount of from 0.005 to 0.2 g/m² being preferred.

The amounts of critical reagents (that is, the 14-crown-4-ether derivative and organic solvents described above) can be readily determined by a skilled worker also. However, the amounts may be within the range of from 0.05 to 2, and preferably from 0.2 to 0.8 g/m², of the 14-crown-4-ether derivative. The organic solvent in the first reagent layer may be present in an amount of from 0.5 to 10, and preferably from 2 to 6, g/m². The organic solvent in the second reagent layer may be present in an amount of from 0.5 to 12, and preferably from 2 to 6, g/m². All amounts are dry weight.

The assay of this invention can be manual or automated. In general, the element is used by physically contacting it with the test specimen (for example from 1 to 200 µl) suspected of containing lithium ion under ambient conditions (although other temperatures can be used). The specimen and reagents become mixed within the layers of the element and any lithium ion complexes with the 14-crown-4-ether derivative to produce a spectrophotometric change as described above. Contact can be achieved in any suitable manner, for example by dipping or immersing the element into the specimen or preferably, by spotting the specimen onto the element by hand, machine or suitable dispensing means.

After specimen application, the element is exposed to any conditioning, such as incubation, heating or otherwise, that may be desirable to quicken or otherwise facilitate obtaining a test result.

Generally within 5 minutes, and preferably within 2.5 minutes, a spectrophotometric measurement is made. This measurement can be made using suitable reflection or transmission spectrophotometric equipment and procedures as a measure of lithium ion concentration in the test sample.

When the preferred 14-crown-4-ether derivatives listed in Table I above are used in the practice of the invention, the spectrophotometric change is generally measured at a wavelength which is within the range of from 400 to 700 nm, with measurement at a wavelength of from 450 to 650 nm being preferred. The use of other complexing compounds may require measurement at other wavelengths as one skilled in the art could readily ascertain.

### Example 1 Preferred Analytical Element for Lithium Ion Determination

The preferred element of this invention and amounts of components are illustrated in the structure:

| Poly(ethylene terephthalate) Support | | |
|---|---|---|
| | | Dry Coverage (g/m²) |
| Spreading Layer | Barium sulfate | 100 |
| | Cellulose acetate | 8 |
| | ESTANE^{TM} polyurethane | 1.4 |
| | TRITON^{TM} X-405 nonionic surfactant | 0.7 |
| Subbing Layer | Poly(vinylpyrrolidone) | 6 |
| | 3-(N-cyclohexylamino)-1-propanesulfonic acid buffer (pH 10.6) | 6 |
| Second Reagent Layer | Hardened gelatin | 10 |
| | SURFLON^{TM} 132 surfactant | 0.02 |
| | 2,4-Di-n-pentylphenol | 3.2 |
| First Reagent Layer | Hardened gelatin | 12 |
| | SURFLON^{TM} 132 surfactant | 0.02 |
| | Compound 2 of Table I | 0.4 |
| | 2,4-Di-n-pentylphenol | 3.2 |

### Example 2 Comparative Example Using Alternative Element

An element similar to that of Example 1 was used to detect lithium ion in a serum specimen and compared to the performance of a Control element which was like it except 2,4-di-n-pentylphenol was omitted from the second reagent layer. The element of this invention was like that of Example 1 except: the polyurethane coverage was 3 g/m², TRITON™ X-100 nonionic surfactant was used in place of TRITON™ X-405 nonionic surfactant, each component of the subbing layer was at 4 g/m², SURFLON™ 145 surfactant was used at 0.01 g/m² instead of SURFLON™ 132, the amount of 2,4-di-n-pentylphenol in the second reagent layer was 4 g/m², and 0.6 g/m² of Compound 2 was used.

The assays were performed on the elements (which had been kept at 25°C and 15% relative humidity until use) as follows:

Serum samples (10 µl) containing varying amounts (0.27 to 10.59 mmolar) of lithium ion were spotted onto the spreading layer of individual elements. After incubation for about 48 seconds at 37°C, the reflectance density (D_{R}) was measured at 600 nm using a standard recording reflectance densitometer. The lithium ion concentrations obtained from these reflectance densities were compared to the predicted concentrations for the various amounts of analyte in the serum samples. Additional densities were measured after about 130 seconds (from specimen spotting) and after about 290 seconds.

Tables II, III and IV below show the reflectance densities measured at 48, 130 and 290 seconds, respectively, for both Control and Example 1 elements. The data include the standard of deviation (S.D.) at each concentration. The standard of deviation is a parameter which indicates the variation from a mean generated from several repeated determinations. It is indicative of precision since a high precision would have a low standard of deviation. The data provided in the 0.27 to 3.71 mmolar concentration is most important since this is the most useful range for determination of lithium ion concentration in patients.

**TABLE II**

| **Reflectance Density (D**_{**n**}**)** | **Actual Lithium Ion Concentration** | **Predicted Lithium Ion Concentration** | **S.D.** |
|---|---|---|---|
| -Control- | | | |
| 0.59038 | 0.27 | 0.30 | 0.0500 |
| 0.60684 | 0.53 | 0.51 | 0.1114 |
| 0.62593 | 0.79 | 0.76 | 0.0568 |
| 0.64594 | 1.02 | 1.01 | 0.0543 |
| 0.68924 | 1.52 | 1.58 | 0.1563 |
| 0.75296 | 2.50 | 2.43 | 0.1221 |
| 0.85375 | 3.72 | 3.81 | 0.2058 |
| 0.93319 | 5.00 | 4.93 | 0.2207 |

| -Example 2- | | | |
|---|---|---|---|
| 0.38294 | 0.27 | 0.31 | 0.0356 |
| 0.40113 | 0.53 | 0.58 | 0.0579 |
| 0.41258 | 0.79 | 0.77 | 0.0631 |
| 0.42807 | 1.02 | 1.02 | 0.0560 |
| 0.45465 | 1.52 | 1.58 | 0.0435 |
| 0.50820 | 2.55 | 2.49 | 0.0452 |
| 0.56291 | 3.71 | 3.64 | 0.0644 |
| 0.62740 | 5.00 | 5.15 | 0.2099 |
| 0.81685 | 10.59 | 10.55 | 0.1966 |
| | | | |

**TABLE III**

| **Reflectance Density (D**_{**n**}**)** | **Actual Lithium Ion Concentration** | **Predicted Lithium Ion Concentration** | **S.D.** |
|---|---|---|---|
| -Control- | | | |
| 0.78156 | 0.27 | 0.31 | 0.0658 |
| 0.80167 | 0.53 | 0.57 | 0.2403 |
| 0.81553 | 0.79 | 0.76 | 0.0886 |
| 0.83239 | 1.02 | 0.98 | 0.1027 |
| 0.87722 | 1.52 | 1.58 | 0.2299 |
| 0.93576 | 2.50 | 2.38 | 0.1232 |
| 1.04067 | 3.72 | 3.82 | 0.2163 |
| 1.11907 | 5.00 | 4.92 | 0.2429 |

| -Example 2- | | | |
|---|---|---|---|
| 0.40299 | 0.27 | 0.32 | 0.0542 |
| 0.42751 | 0.53 | 0.55 | 0.0516 |
| 0.45080 | 0.79 | 0.79 | 0.0548 |
| 0.47036 | 1.02 | 1.00 | 0.0417 |
| 0.51114 | 1.52 | 1.47 | 0.0593 |
| 0.59127 | 2.55 | 2.51 | 0.0580 |
| 0.66887 | 3.71 | 3.66 | 0.1363 |
| 0.75481 | 5.00 | 5.12 | 0.2172 |
| 1.01130 | 10.59 | 10.55 | 0.1998 |

**TABLE IV**

| **Reflectance Density (D**_{**n**}**)** | **Actual Lithium Ion Concentration** | **Predicted Lithium Ion Concentration** | **S.D.** |
|---|---|---|---|
| -Control- | | | |
| 1.01963 | 0.27 | 0.34 | 0.0964 |
| 1.04389 | 0.53 | 0.71 | 0.4242 |
| 1.04574 | 0.79 | 0.74 | 0.1413 |
| 1.05871 | 1.02 | 0.94 | 0.1240 |
| 1.10091 | 1.52 | 1.61 | 0.3912 |
| 1.14445 | 2.50 | 2.30 | 0.1471 |
| 1.23757 | 3.72 | 3.81 | 0.3548 |
| 1.30090 | 5.00 | 4.87 | 0.3579 |

| -Example 2- | | | |
|---|---|---|---|
| 0.39812 | 0.27 | 0.32 | 0.0480 |
| 0.42333 | 0.53 | 0.55 | 0.0518 |
| 0.44727 | 0.79 | 0.79 | 0.0541 |
| 0.46905 | 1.02 | 1.01 | 0.0335 |
| 0.51070 | 1.52 | 1.47 | 0.0457 |
| 0.59200 | 2.55 | 2.50 | 0.0542 |
| 0.67216 | 3.71 | 3.66 | 0.1163 |
| 0.76110 | 5.00 | 5.13 | 0.1737 |
| 1.02234 | 10.59 | 10.55 | 0.1798 |

The data in these tables indicate that placing the aromatic organic solvent in the second reagent layer improves the precision of the assay particularly at the longer evaluation times (130 and 290 seconds) and in the desired concentration range of 0.27 to 3.71 mmolar. This enables a user of the element to accurately determine lithium ion concentration at longer times.

### Examples 3 & 4 Elements Having Various Amounts of Organic Solvent in Second Reagent Layer

Elements similar to that shown in Example 2 were prepared having 1 and 5 g/m² of 2,4-di-n-pentylphenol in the second reagent layer (Examples 3 and 4, respectively), and used to detect various levels of lithium ion (0.27-10.59 mmolar) according to the procedure described in Example 2. Precision was acceptable at each concentration of organic solvent, as shown with the data presented in Tables V (Example 3) and VI (Example 4) below.

**TABLE V**

| **Reflectance Density (D**_{**n**}**)** | **Actual Lithium Ion Concentration** | **Predicted Lithium Ion Concentration** | **S.D.** |
|---|---|---|---|
| -48 seconds- | | | |
| 0.43183 | 0.27 | 0.35 | 0.0164 |
| 0.45324 | 0.53 | 0.54 | 0.0282 |
| 0.47795 | 0.79 | 0.78 | 0.0430 |
| 0.49758 | 1.02 | 0.99 | 0.0400 |
| 0.53201 | 1.52 | 1.41 | 0.0597 |
| 0.61064 | 2.55 | 2.56 | 0.0989 |
| 0.68539 | 3.71 | 3.92 | 0.0574 |
| 0.73044 | 5.00 | 4.87 | 0.6131 |
| 0.94355 | 10.59 | 10.55 | 0.0630 |

| -130 seconds- | | | |
|---|---|---|---|
| 0.44875 | 0.27 | 0.32 | 0.0189 |
| 0.47573 | 0.53 | 0.54 | 0.0298 |
| 0.50347 | 0.79 | 0.78 | 0.0431 |
| 0.52751 | 1.02 | 1.01 | 0.0451 |
| 0.56852 | 1.52 | 1.43 | 0.0555 |
| 0.66064 | 2.55 | 2.55 | 0.0787 |
| 0.75085 | 3.71 | 3.86 | 0.0370 |
| 0.81433 | 5.00 | 4.92 | 0.4491 |
| 1.08263 | 10.59 | 10.57 | 0.1380 |

| -290 seconds- | | | |
|---|---|---|---|
| 0.44499 | 0.27 | 0.31 | 0.0152 |
| 0.47193 | 0.53 | 0.54 | 0.0309 |
| 0.49855 | 0.79 | 0.79 | 0.0463 |
| 0.52136 | 1.02 | 1.01 | 0.0458 |
| 0.56143 | 1.52 | 1.44 | 0.0567 |
| 0.65089 | 2.55 | 2.56 | 0.0786 |
| 0.73822 | 3.71 | 3.85 | 0.0392 |
| 0.80076 | 5.00 | 4.91 | 0.4259 |
| 1.06657 | 10.59 | 10.57 | 0.1352 |
| | | | |

**TABLE VI**

| **Reflectance Density (D**_{**n**}**)** | **Actual Lithium Ion Concentration** | **Predicted Lithium Ion Concentration** | **S.D.** |
|---|---|---|---|
| -48 seconds- | | | |
| 0.42546 | 0.27 | 0.31 | 0.0221 |
| 0.45121 | 0.53 | 0.54 | 0.0277 |
| 0.47606 | 0.79 | 0.78 | 0.0450 |
| 0.49740 | 1.02 | 0.99 | 0.0096 |
| 0.54326 | 1.52 | 1.49 | 0.0560 |
| 0.62680 | 2.55 | 2.53 | 0.0400 |
| 0.70933 | 3.71 | 3.72 | 0.0605 |
| 0.79057 | 5.00 | 5.05 | 0.1284 |
| 1.05804 | 10.59 | 10.57 | 0.1515 |

| -130 seconds- | | | |
|---|---|---|---|
| 0.41903 | 0.27 | 0.32 | 0.0246 |
| 0.44748 | 0.53 | 0.55 | 0.0310 |
| 0.47560 | 0.79 | 0.79 | 0.0534 |
| 0.49669 | 1.02 | 0.98 | 0.0156 |
| 0.54473 | 1.52 | 1.45 | 0.0526 |
| 0.64198 | 2.55 | 2.55 | 0.0431 |
| 0.72740 | 3.71 | 3.67 | 0.0354 |
| 0.82192 | 5.00 | 5.09 | 0.1384 |
| 1.11149 | 10.59 | 10.56 | 0.1590 |

| -290 seconds- | | | |
|---|---|---|---|
| 0.41180 | 0.27 | 0.33 | 0.0254 |
| 0.43846 | 0.53 | 0.55 | 0.0329 |
| 0.46573 | 0.79 | 0.79 | 0.0575 |
| 0.48559 | 1.02 | 0.98 | 0.0161 |
| 0.53145 | 1.52 | 1.45 | 0.0534 |
| 0.62512 | 2.55 | 2.54 | 0.0499 |
| 0.70829 | 3.71 | 3.67 | 0.0254 |
| 0.80133 | 5.00 | 5.10 | 0.1465 |
| 1.08220 | 10.59 | 10.56 | 0.1270 |

### Example 5 Comparison of Calibration Curves

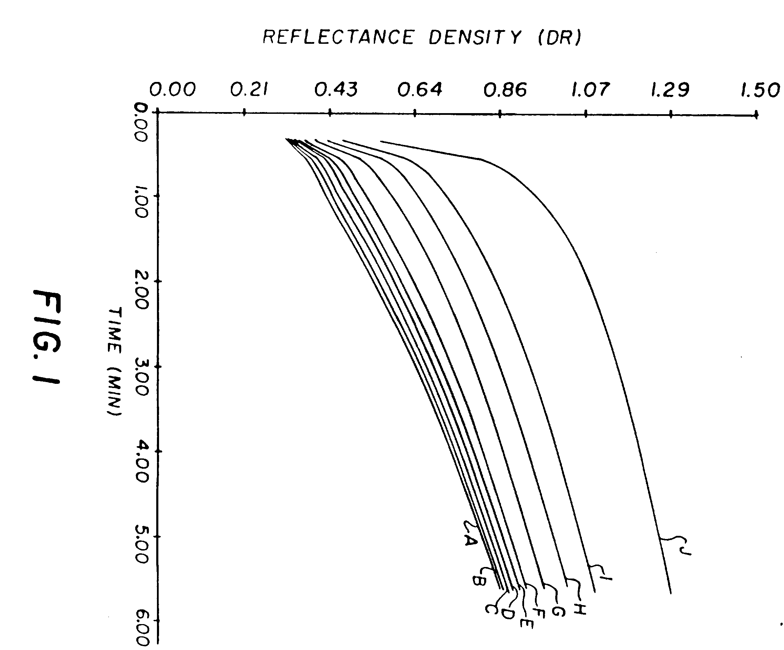

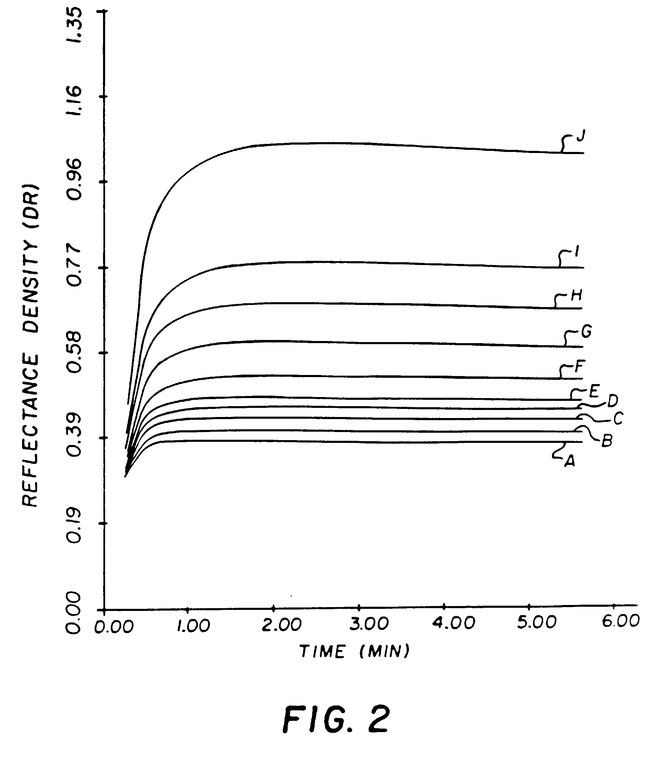

This example shows a comparison of calibration curves generated at various concentrations (0.27-10.59 mmolar) of lithium ion using an element of this invention and a Control element which is outside the scope of this invention. The element of this invention was that shown in Example 1 and the Control element was similarly prepared except that the organic solvent was omitted from the second reagent layer. The procedure for determining reflectance density was like that described in Example 2. FIGS. 1 and 2 show the calibration curves for various analyte concentrations over time for the assays using the Control (FIG. 1) and invention (FIG. 2), respectively.

The amounts of lithium ion concentration used in these assays were (as defined in the FIGURES):
Fluid A: water (no lithium ions),
Fluid B: 0.27 mmolar,
Fluid C: 0.53 mmolar,
Fluid D: 0.79 mmolar,
Fluid E: 1.02 mmolar,
Fluid F: 1.52 mmolar,
Fluid G: 2.50 mmolar,
Fluid H: 3.72 mmolar,
Fluid I: 3.72 mmolar, and
Fluid J: 5.00 mmolar.

### Example 6 Effects of Fluorinated Surfactants

This example demonstrates the importance of having perfluoroalkyl surfactants in the second reagent layer with the organic solvent to stabilize the suspension of solvent in the coating formulation.

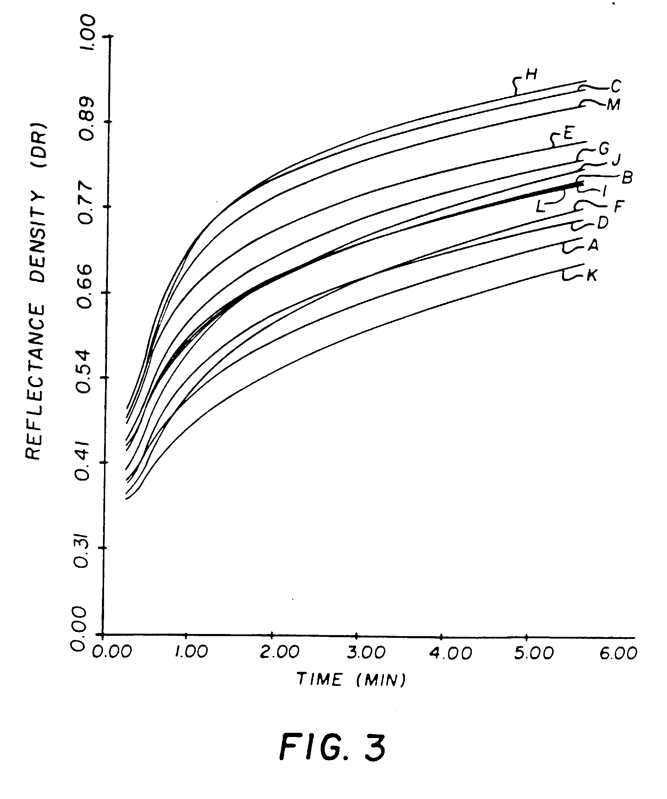

The element of Example 1 was used to assay samples of various fluids containing various amounts (0- 2.4 mmolar) of lithium ion. A Control element was similarly prepared except that the SURFLON^{TM} 132 surfactant was replaced in both reagent layers with ALKANOL^{TM} XC nonionic surfactant (0.02 g/m²). The results are illustrated in FIGURES 3 (Control) and 4 (Invention). The fluids used were as followed:
Fluid A: Pool of patient samples, 0.05 mmolar lithium ion,
Fluid B: Pool of patient samples, 0.9 mmolar lithium ion,
Fluid C: Pool of patient samples, 2.4 mmolar lithium ion,
Fluid D: 0.55 mmolar lithium ion,
Fluid E: 1.6 mmolar lithium ion,
Fluid F: Pool of patient samples, 0.23 mmolar lithium ion,
Fluid G: Pool of patient samples, 1.05 mmolar lithium ion,
Fluid H: Pool of patient samples, 2.47 mmolar lithium ion,
Fluid I: Control fluid, 0.71 mmolar lithium ion,
Fluid J: Control fluid, 0.82 mmolar lithium ion,
Fluid K: Pool of patient samples, no lithium ion,
Fluid L: Pool of patient samples, 0.89 mmolar lithium ion,
Fluid M: Pool of patient samples, 2.36 mmolar lithium ion.

It is clear that the element of this invention showed a reduced rate of background dye development (that is, dye development from sources other than lithium ion) compared to the Control element.

## Claims

1. A dry multilayer analytical element for the determination of lithium ion comprising a support having thereon, in order and in fluid contact:
a) a first reagent layer containing a 14-crown-4-ether derivative dye for lithium ion,
b) a second reagent layer containing a perfluoroalkyl surfactant, and an aromatic phenol organic solvent having the structure (I): 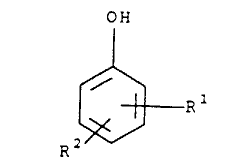 wherein R¹ and R² are independently alkyl of 1 to 20 carbon atoms or acyl of 2 to 20 carbon atoms, and
c) a porous spreading layer,
the element further comprising, in a layer other than the first or second reagent layers, a buffer present in an amount sufficient to maintain a pH in the element of at least 9 during an assay,
the element characterized in that it further comprises a second aromatic phenol organic solvent in the first reagent layer, which second aromatic phenol organic solvent has the structure (I), and the first and second organic solvents can be the same or different and in that the 14-crown-4-ether derivative dye for lithium ion is absent from the second reagent layer.

2. The element as claimed in claim 1 further comprising a subbing layer between the second reagent and porous spreading layers, and wherein the buffer is in the subbing layer.

3. The element as claimed in either of claims 1 or 2 wherein the buffer is present in an amount sufficient to maintain a pH in the element of at least 10 during an assay.

4. The element as claimed in any of claims 1 to 3 wherein the buffer is 3-(cyclohexylamino)-1-propanesulfonic acid, 4-aminobutyric acid, phosphate or arginine.

5. The element as claimed in any of claims 1 to 4 wherein the 14-crown-4-ether derivative dye has the structure (II): 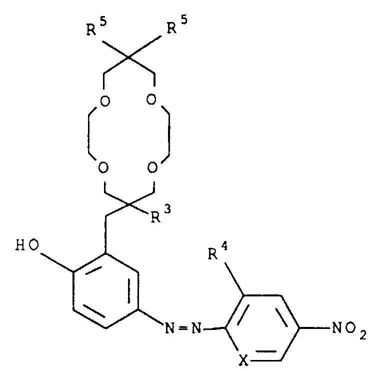 wherein R³ is substituted or unsubstituted alkyl of 1 to 15 carbon atoms, R⁴ is hydrogen, nitro, methylsulfonyl, N-alkylsulfamoyl or N,N-dialkylsulfamoyl wherein said alkyl is substituted or unsubstituted and has 1 to 20 carbon atoms, R⁵ is substituted or unsubstituted alkyl of 1 to 10 carbon atoms, and X is N or CH.

6. The element as claimed in any of claims 1 to 5 wherein the first and second aromatic phenol organic solvents are the same.

7. The element as claimed in any of claims 1 to 6 wherein R¹ and R² are the same and are butyl, pentyl or hexyl.

8. The element as claimed in any of claims 1 to 7 wherein the first and second aromatic phenol organic solvents are both either 2,4-di-n-pentylphenol or 2,4-di-t-pentylphenol.

9. The element as claimed in any of claims 1 to 8 wherein both of the reagent layers contain the same perfluoroalkyl surfactant.

10. A method for the determination of lithium ion comprising:
A. contacting a liquid sample suspected of containing lithium ion with a dry multilayer analytical element as claimed in any of claims 1 to 9, and
B. determining the change in spectrophotometric signal in the element from complexation of the 14-crown-4-ether dye derivative with lithium ion in the liquid sample.

## Patentansprüche

1. Trockenes mehrschichtiges analytisches Element zur Bestimmung von Lithiumionen, das einen Träger umfaßt, der darauf, in Abfolge und in Fließkontakt:
a) eine erste Reagens-Schicht, die einen 14-Krone-4-Ether-Derivat-Farbstoff für Lithiumionen enthält,
b) eine zweite Reagens-Schicht, die eine oberflächenaktive Perfluoralkylverbindung und ein organisches Lösungsmittel auf der Basis eines aromatischen Phenols mit der Struktur (I) enthält: 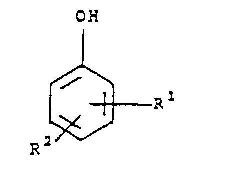 in der R¹ und R² unabhängig Alkyl mit 1 bis 20 Kohlenstoffatomen oder Acyl mit 2 bis 20 Kohlenstoffatomen sind, und
c) eine poröse Verteilschicht,
umfaßt, wobei das Element außerdem, in einer anderen Schicht als der ersten oder der zweiten Reagensschicht, einen Puffer umfaßt, der in einer Menge vorliegt, die ausreichend ist, um während eines Tests einen pH im Element von wenigstens 9 aufrechtzuerhalten,
wobei das Element dadurch gekennzeichnet ist, daß es außerdem als zweites organisches Lösungsmittel auf der Basis eines aromatischen Phenols in der ersten Reagens-Schicht umfaßt, wobei das organische Lösungsmittel auf der Basis von aromatischem Phenol die Struktur (I) aufweist und das erste und das zweite organische Lösungsmittel identisch oder verschieden sein können, und daß der 14-Krone-4-Ether-Derivat-Farbstoff für Lithiumionen in der zweiten Reagens-Schicht nicht vorhanden ist.

2. Element nach Anspruch 1, weiter gekennzeichnet durch eine Hilfsschicht zwischen der zweiten Reagens-Schicht und der porösen Verteilschicht, und daß der Puffer in der Hilfsschicht vorliegt.

3. Element nach Anspruch 1 oder 2, dadurch gekennzeichnet, daß der Puffer in einer Menge vorliegt, die ausreichend ist, um während eines Tests einen pH im Element von wenigstens 10 aufrechtzuerhalten.

4. Element nach einem der Ansprüche 1 bis 3, dadurch gekennzeichnet, daß der Puffer 3-(Cyclohexylamino)-1-propansulfonsäure, 4-Aminobuttersäure, Phosphat oder Arginin ist.

5. Element nach einem der Ansprüche 1 bis 4, dadurch gekennzeichnet, daß der 14-Krone-4-Ether-Derivat-Farbstoff die Struktur (II) aufweist: 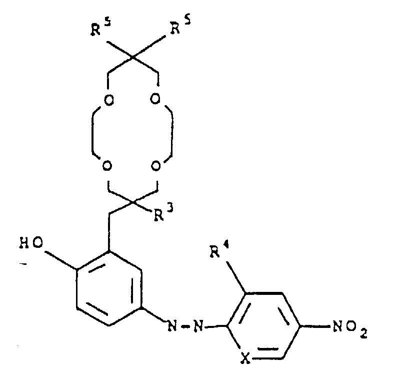 in der R³ substituiertes oder unsubstituiertes Alkyl mit 1 bis 15 Kohlenstoffatomen ist, R⁴ Wasserstoff, Nitro, Methylsulfonyl, N-Alkylsulfamoyl oder N,N-Dialkylsulfamoyl ist, wobei das Alkyl substituiert oder unsubstituiert ist und 1 bis 20 Kohlenstoffatome aufweist, R⁵ substituiertes oder unsubstituiertes Alkyl mit 1 bis 10 Kohlenstoffatomen ist und X N oder CH ist.

6. Element nach einem der Ansprüche 1 bis 5, dadurch gekennzeichnet, daß das erste und das zweite organische Lösungsmittel auf der Basis von aromatischem Phenol identisch sind.

7. Element nach einem der Ansprüche 1 bis 6, dadurch gekennzeichnet, daß R¹ und R² identisch und Butyl, Pentyl oder Hexyl sind.

8. Element nach einem der Ansprüche 1 bis 7, dadurch gekennzeichnet, daß das erste und das zweite organische Lösungsmittel auf der Basis von aromatischem Phenol beide entweder 2,4-Di-n-pentylphenol oder 2,4-Ditert.-pentylphenol sind.

9. Element nach einem der Ansprüche 1 bis 8, dadurch gekennzeichnet, daß beide Reagens-Schichten dieselbe oberflächenaktive Perfluoralkylverbindung enthalten.

10. Verfahren zur Bestimmung von Lithiumionen, welches umfaßt:
A. In-Kontakt-Bringen einer flüssigen Probe, die im Verdacht steht, Lithiumionen zu enthalten, mit einem trockenen mehrschichtigen analytischen Element nach einem der Ansprüche 1 bis 9 und
B. Bestimmen der Veränderung des spektrophotometrischen Signals im Element aus der Komplexierung des 14-Krone-4-Ether-Farbstoff-Derivats mit Lithiumionen in der flüssigen Probe.

## Revendications

1. Un élément sec multicouche analytique pour la détermination de l'ion lithium, comprenant un support y prévu, dans l'ordre et en contact avec le fluide :
a) une première couche de réactif contenant un colorant du type dérivé d'un éther 14-couronne-4 (14 crown-4-ether) pour l'ion lithium,
b) une seconde couche de réactif contenant un agent tensioactif du type per-fluoroalkyle, et un solvant organique du type phénol aromatique ayant la structure (I): 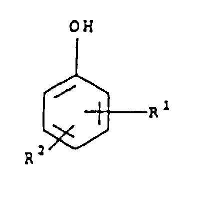 dans laquelle R¹ et R² représentent indépendamment un alkyle de 1 à 20 atomes de carbone ou un acyle de 2 à 20 atomes de carbone, et
c) une couche d'étalement poreuse,
l'élément comprenant en outre, dans une couche différente des première ou seconde couches de réactif, un agent tampon dans une quantité suffisante pour maintenir un pH, dans l'élément, d'au moins 9 pendant un essai,
l'élément étant caractérisé en ce qu'il comprend en outre un second solvant organique du type phénol aromatique dans la première couche de réactif, ce second solvant organique de type phénol aromatique présentant la structure (I), tandis que les premier et second solvants organiques peuvent être identiques ou différents et que le colorant du type dérivé d'éther 14-couronne-4 (14 crown-4-ether) pour l'ion lithium est absent de la seconde couche de réactif.

2. L'élément tel que revendiqué dans la revendication 1, comprenant en outre une sous-couche entre la couche de second réactif et la couche d'étalement poreuse, et dans lequel l'agent tampon se trouve dans la sous-couche.

3. L'élément tel que revendiqué dans l'une quelconque des revendications 1 ou 2, dans lequel l'agent tampon est présent en une quantité suffisante pour maintenir un pH, dans l'élément, d'au moins 10 pendant un essai.

4. L'élément tel que revendiqué dans l'une quelconque des revendications 1 à 3, dans lequel l'agent tampon est l'acide 3-(cyclohexylamino)-1-propanesulfonique, l'acide 4-aminobutyrique, un phosphate ou l'arginine.

5. L'élément tel que revendiqué dans l'une quelconque des revendications 1 à 4, dans lequel le colorant de type dérivé d'éther 14-couronne-4 (14 crown-4-ether) présente la structure (II) : 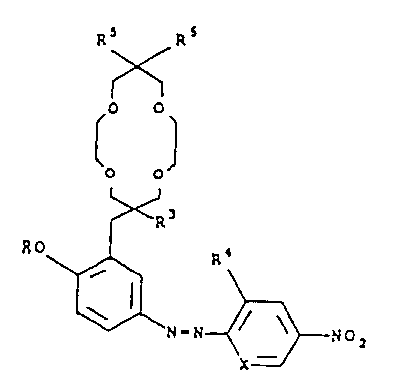 dans laquelle
◆ R³ est un alkyle, substitué ou non substitué, de 1 à 15 atomes de carbone,
◆ R⁴ est de l'hydrogène, un radical nitro, méthylsulfonyle, N-alkylsulfamoyle ou N,N-dialkylsulfamoyle, dans lequel ledit alkyle est substitué ou non substitué et possède de 1 à 20 atomes de carbone,
◆ R⁵ est un alkyle substitué ou non substitué de 1 à 10 atomes de carbone, et
◆ X est N ou CH.

6. L'élément tel que revendiqué dans l'une quelconque des revendications 1 à 5, dans lequel les premier et second solvants organiques de type phénol aromatique sont identiques.

7. L'élément tel que revendiqué dans l'une quelconque des revendications 1 à 6, dans lequel R¹ et R² sont identiques et sont un radical butyle, pentyle ou hexyle.

8. L'élément tel que revendiqué dans l'une quelconque des revendications 1 à 7, dans lequel les premier et second solvants organiques de type non-aromatique sont, tous les deux, soit du 2,4-di-n-pentylphénol ou du 2,4-di-r-pentylphénol.

9. L'élément tel que revendiqué dans l'une quelconque des revendications 1 à 8, dans lequel les deux couches de réactif renferment le même agent tensioactif de type perfluoroalkyle.

10. Un procédé pour la détermination de l'ion lithium comportant :
A. la mise en contact d'un échantillon liquide suspecté de renfermer de l'ion lithium avec un élément sec multicouche analytique tel que revendiqué dans l'une quelconque des revendications 1 à 9, et
B. la détermination de la modification du signal spectrophotométrique dans l'élément à partir de la complexation du dérivé de type colorant d'éther 14-couronne-4 (14 crown-4-ether) avec l'ion lithium dans l'échantillon liquide.
